(19) **Europäisches Patentamt / European Patent Office / Office européen des brevets**

(11) **EP 3 469 097 B1**

(12) **EUROPEAN PATENT SPECIFICATION**

(45) Date of publication and mention
of the grant of the patent:
**19.02.2020 Bulletin 2020/08**

(21) Application number: **17729151.5**

(22) Date of filing: **14.06.2017**

(51) Int Cl.:
*C12Q 1/6834* $^{(2018.01)}$    *C12N 15/10* $^{(2006.01)}$

(86) International application number:
**PCT/EP2017/064509**

(87) International publication number:
**WO 2017/216211 (21.12.2017 Gazette 2017/51)**

(54) **METHOD FOR THE SEPARATION OF A MODIFIED POLYNUCLEOTIDE**

VERFAHREN ZUR ABTRENNUNG EINES MODIFIZIERTEN POLYNUKLEOTIDS

PROCÉDÉ DE SÉPARATION D'UN POLYNUCLÉOTIDE MODIFIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.06.2016 EP 16174455**

(43) Date of publication of application:
**17.04.2019 Bulletin 2019/16**

(73) Proprietor: **Base4 Innovation Limited
Cambridge, Cambridgeshire CB3 0FA (GB)**

(72) Inventor: **DEAR, Paul
Cambridge
Cambridgeshire CB3 0FA (GB)**

(74) Representative: **Lau, Sarah Jane
Kilburn & Strode LLP
Lacon London
84 Theobalds Road
London WC1X 8NL (GB)**

(56) References cited:
**EP-A1- 2 196 544         WO-A1-01/27328
WO-A2-2005/003375    US-A1- 2010 311 602
US-A1- 2013 122 485   US-A1- 2014 335 528**

**Description**

This invention relates to a method of isolating a modified polynucleotide from a crude mixture of modified polynucleotides.

For many biotechnological applications, including genetic sequencing, it is often desirable that a given polynucleotide fragment P should have different functional groups, sequence elements, structures or moieties at its two ends. In other words if these moieties are denoted generally as A and B, fragments of the form A-P-B are preferred. One simple and convenient way to prepare such modified polynucleotides is to start with the unmodified polynucleotide, and label it in such a way that either end is equally likely to be labelled with either A or B. In this case, then approximately half of the molecules will be labelled with the same moiety at each (i.e. A-P-A or B-P-B,) whilst about half will be the desired differently modified A-P-B types. It is then necessary to isolate the desired fragments from the A-P-A or B-P-B fragments.

One way to accomplish this separation is to choose the moieties A and B such that each may be bound to a correspondingly modified solid support, and that the binding of A is orthogonal to the binding of B (that is, a support is chosen which binds A but not B, or vice versa). Using this orthogonal binding, the mixed sample can then be purified to enrich for A-P-B molecules, in ways well-known to one of skill in the art. For example, the mixture can be first be brought into contact with a solid support which binds moiety A. Unbound molecules (of the type B-P-B) are then washed away and discarded. Thereafter, the bound molecules (A-P-A and A-P-B) are released from the solid support and the recovered fraction then brought into contact with a solid support which binds moiety B. This then enables unbound molecules (A-P-A) to be washed away. Finally, the molecules bound to this second support (which will be of type A-P-B) can be released and recovered.

One problem with this approach is that it can be very cumbersome. For example, it can be difficult to bind the moieties A and B strongly whilst also being able to release them when required. The need for multiple heterogeneous handling steps can also be problematic when small amounts of sample are used.

J. Nanotechnol. (2016) 7 138-148 describes a method in which DNA tethered to beads by means of a protein handle can be manipulated by optical tweezers. However this method can be cumbersome to operate on a large scale.

WO 01/27328 and US2014/335528 describe multi-step methods for separating polynucleotides using beads. However neither teaches the method of the present application.

EP 2196544 teaches a kit for detecting a target nucleic acid comprised of first and second beads, labels and probes and wherein at least one of the bead types is magnetic.

A separation method has now been developed which overcomes these problems by using at least two easily manipulated bead types which differ in their physical properties as well as in their ability to bind moieties A and B. Thus according to the present disclosure there is provided a method of isolating a modified polynucleotide having the formula A-P-B from a crude mixture of modified polynucleotides also including those having the formulae A-P-A and B-P-B, wherein P is a polynucleotide region and A and B are different modifying moieties or nucleotide sequences, characterised by the steps of (a) reacting the crude mixture concurrently or consecutively with beads of one type capable of binding to moiety A but not B, and beads of a second type capable of binding to moiety B but not A, the two types of bead differing in one or more of their sizes, densities, paramagnetic properties or optical properties to produce an intermediate product; (b) fractionating the intermediate product based on the properties of each type of bead and of pairs of different types of bead conjoined by A-P-B polynucleotides, such that only or predominantly conjoined pairs of the two different types of bead are retained and (c) optionally, releasing one or both beads from such conjoined pairs such that the polynucleotide may be recovered.

In preferred arrangements of the present disclosure, the different bead types are characterised by having differing densities and the method involves, at least in part, fractionation based on the associated density difference. Thus, in a first aspect of the present invention, there is provided a method of isolating a modified polynucleotide having the formula A-P-B from a crude mixture of modified polynucleotides also including those having the formulae A-P-A and B-P-B, wherein P is a polynucleotide region and A and B are different modifying moieties or nucleotide sequences wherein step (a) comprises reacting the crude mixture with first beads modified with complementary moieties A' capable of binding to A and second beads having a density lower than that of the first beads and modified with complementary moieties B' capable of binding to B to produce an intermediate product comprised of first-second bead-pairs connected by a modified polynucleotide together with one or more of unused or unpaired first and second beads and corresponding first-first, second-second bead-pairs; step (b) comprises separating the first-second bead-pairs and optionally any unused or unpaired second beads or second-second bead-pairs from the intermediate product in an aqueous medium having a density greater than that of the first-second beads-pairs but less than that of the first beads and step (c) comprises thereafter separating the first-second bead-pairs from the product of step (b) in an aqueous medium having a density less than that of the first-second bead-pairs but greater than that of the second beads.

As used herein and when applied to the bead pairs conjoined by the modified polynucleotide, the term first-first bead pair means two first beads connected by the modified polynucleotide of formula A-P-A; the term second-second bead pair means two second beads connected by the modified polynucleotide of formula B-P-B and the term first-second bead pair one bead of each type connected by a modified polynucleotide of formula A-P-B.

Regarding step (a) of the invention, the intermediate product is preferably created in two sub-steps by first reacting the crude mixture with the first beads and then with the second beads. In such an embodiment the beads may optionally be washed between this first and second sub-steps to remove any B-P-B molecules from the system. If this is done, there will be few or no second-second bead pairs produced in the second sub-step. In another embodiment, these sub-steps are reversed so that the crude mixture is first reacted with the second beads and then the first beads. In yet another, the crude mixture is treated with both bead types at the same time.

As further explained below, the modified polynucleotides employed can themselves be polynucleotides having characteristic A and B sequence regions at their ends. Alternatively only the region P may be a polynucleotide with the flanking regions being other moieties. The polynucleotides themselves may be selected from the group consisting of poly-ribonucleotides, poly-deoxyribonucleotides (including but not limited to those derived from naturally-occurring genetic material) or synthetic versions thereof including not only those derived from the five nucleobases found in nature but also one on more nucleobases which are of man-made origin.

By the term bead is meant a discrete unit of solid material which is insoluble in any reaction medium employed and which can be manipulated therein. Typically such beads will be spherical or substantially spherical and made of a polymer, metal or metal oxide. In one embodiment the bead will have a diameter in the range 10nm to 10mm; preferably in the range 0.1 to 50 $\mu$m.

In the various aspects of the invention taught herein, the first beads are modified with complementary moieties A' and are denser than the second beads. In one embodiment they have a density of greater than $1.5g/cm^3$ preferably in the range from 1.5 to $2.0g/cm^3$. The second beads which are modified with complementary moieties B' suitably have a density in the range up to 1.5 preferably in the range from 1.05 to 1.25 $g/cm^3$. Preferably, there is at least a $0.25g/cm^3$, more preferably $0.5g/cm^3$ difference between the densities of the first and second beads.

Moieties A' and B' are chosen to be complementary with the modifying moieties A and B of the various modified polynucleotides in the crude mixture thereby allowing complexes comprising complementary pairs A/A' and B/B' to be formed. In one embodiment, either or both of these complementary pairs may be comprised of complementary oligonucleotide regions with A and B simply being characteristic RNA or DNA regions on a polynucleotide analyte. In another, at least one of the complementary pairs will for example comprise either (1) a protein/substrate pair such as streptavidin or avidin with biotin or (2) an antibody/antigen pair such as anti-digoxigenin and digoxigenin. These types of complementary pair have the advantage that both bead types can be easily surface-modified to attach one or other of the moieties comprising the pair. They also have the advantage that any binding or complexing between the elements of the pair is reversible. However the choice of the components of the complementary pair is often not critical and they can equally be comprised of other moiety pairs which react together to form a labile ionic or covalent bond. With this in mind, other possible complementary pairs will become readily apparent to one skilled in the art as will the methods of attaching the relevant parts to both the polynucleotide and the beads. In one preferred embodiment, the polynucleotides are modified with biotin and digoxigenin, the first beads labelled with streptavidin and the second beads with anti-digoxigenin. In another embodiment the labels on the first and second beads are reversed.

In one preferred embodiment the first beads are magnetic and for example may comprise a paramagnetic core encapsulated by a dielectric polymer such as polystyrene which is functionalised with A'; as is the case for those beads sold under the name Dynal®. The second beads may, for example, comprise low density or expanded polystyrene spheres having B' functionality.

In step (a) of the method the crude mixture is brought into contact with the first and second beads to create bead-pairs tethered to each other by means of a bridging modified polynucleotide molecule. Depending on the nature of the particular modified polynucleotide molecule in the crude mixture these bead-pairs are hereinafter called first-first bead-pairs, second-second bead-pairs or first-second bead-pairs. In one embodiment the number ratio of first to second beads used in step (a) is in the range 0.75 to 1.5; in another it is greater than or equal to 1. Suitably, the total number of modified polynucleotide molecules in the crude mixture is chosen so that the number ratio of such molecules to total beads is relatively low, for example in the range 0.001 to 2, preferably 0.001 to 1, and most preferably in the range 0.001 to 0.5. If one of these lower ratios is employed, relatively few beads will encounter and bind to a modified polynucleotide molecule; and even fewer will encounter and bind to more than one such molecule. Conversely, each modified polynucleotide molecule will eventually encounter and bind to both types of beads after a period of incubation.

For many applications it is preferred that only bead-pairs carrying a single modified polynucleotide molecule with two different ends (A-P-B) are ultimately recovered, with few or none of the recovered bead-pairs carrying two or more of such molecules. Here, if the number ratio of modified polynucleotide molecules to total beads is extremely low (for example, 0.00001 to 1), then essentially all of the recovered bead-pairs will be of this type since no bead will encounter and bind to more than one modified polynucleotide. However, as the number ratio of modified polynucleotide molecules to beads is increased, a proportion of beads will encounter and bind to two or more modified polynucleotide molecules, making it possible that these less desirable bead pairs will be included in the recovered fraction. The expected proportions of these other types may be calculated using Poisson statistics, assuming random encounters between all the components of the mixture. They are set out in the following Table:

| Number ratio of modified polynucleotide molecules (P) to beads | % of empty beads | % of 1 P per bead | % of >1 P per bead |
|---|---|---|---|
| 5 | 0.674 | 3.369 | 95.957 |
| 1 | 36.788 | 36.788 | 26.424 |
| 0.5 | 60.653 | 30.327 | 9.02 |
| 0.2 | 81.873 | 16.375 | 1.752 |
| 0.1 | 90.484 | 9.048 | 0.468 |
| 0.05 | 95.123 | 4.756 | 0.121 |
| 0.01 | 99.005 | 0.99 | 0.005 |

Step (a) and its component sub-steps are, in one embodiment, carried out in a liquid medium, suitably an aqueous medium, at a temperature in the range 10 to 40°C. In another embodiment, the intermediate product is washed before step (b) is carried out to remove any residual modified polynucleotide molecules.

In one embodiment, in step (b) the intermediate product formed in step (a) is separated into two fractions in a liquid medium, preferably an aqueous medium, having a density more than that of the first-second bead-pairs. Preferably, this separation is carried out in an aqueous medium and in one embodiment is achieved by simply adjusting the density of the medium employed in step (a). Alternatively, the solid components of the intermediate product can be separated from the associated medium by filtration (or by centrifugation, assuming that the medium of step (a) is of lower density than either bead type) and a fresh liquid medium of the correct density employed. In one embodiment, modification of the density of this liquid medium is achieved through adding and/or controlling the concentration of a soluble Group IA or IIA salt for example a sodium, potassium or caesium halide. In another it is achieved by adding and/or controlling the concentration of a sugar in the liquid medium.

The exact density of the liquid medium required to carry out this step is related to the density of the first-second bead pairs which in turn is a function of the sizes and densities of the first and second beads themselves. For example, where the first and second beads are the same size, the density of the first-second bead-pairs will to a first approximation be the average of the densities of the two bead types since for relatively short lengths of modified polynucleotides (up to say 50,000 nucleotides) the density effect of this component can be ignored. Thus where the first beads have a density of $1.8 \text{g/cm}^3$ and the second beads have a density of $1.05 \text{g/cm}^3$ the density of the first-second bead pairs will be approximately $1.45 \text{g/cm}^3$ and the corresponding liquid medium will be denser than this. Where the sizes of the first and second beads are different, the density of the first-second bead pairs may be calculated using the general relationship:

$$D_{(first-second)} = (V_{First} \cdot D_{First} + V_{Second} \cdot D_{Second})/(V_{first} + V_{Second})$$

where D and V respectively represent the density and volume of the relevant component. In the case where the beads are spheres this relationship becomes:

$$D_{(first-second)} = (R^3_{First} \cdot D_{First} + R^3_{Second} \cdot D_{Second})/(R^3_{first} + R^3_{Second})$$

where R is the radius of the respective bead.

Preferably the density of the liquid medium should be in the range 100-120% of this density of the first-second bead pairs. In one preferred embodiment, the beads are substantially spherical and the second beads are larger than the first

The net effect of using a liquid medium of this density in step (b) is that any unused or unpaired first beads together with the first-first bead-pairs will tend to sink whilst the others (unused or unpaired second beads, any second-second bead pairs and the first-second bead pairs) will tend to float. This fractionation process can be accelerated by, for example, by rapid stirring if the volumes are large or centrifuging of the components of this step. Thereafter, the bottom and top fractions may be separated by conventional means and the beads contained in the bottom fraction discarded if so desired. If the first beads are magnetic the components which sink will have a tendency to clump in the presence of a magnetic field making pellets which are easy to remove.

In one embodiment of step (c) of the process, those beads contained in the top fraction are then treated with a liquid medium, preferably an aqueous medium, having a density less than that of the first-second bead pairs but greater than that of the second beads. Again, as explained above, this can be achieved either by adjusting the density of the liquid medium or by use of a fresh liquid medium after filtration. Preferably the density of this liquid medium is in the range 75-90% of the density of the first-second bead pairs and considerations similar to those discussed above apply. At this lower density the first-second bead pairs now tend to sink whilst the remaining beads will still float meaning that the first-second bead pairs can be separated by a second fractionation. Once again, this fractionation process can be accelerated by the use of a centrifuge. In one preferred embodiment, where the first beads are magnetic this process can be further accelerated by applying a magnetic field, e.g. from an electromagnet, to the walls of any vessel containing the components of step (c). In this case, the first-second beads being the only remaining magnetic component will be drawn away from the others thereby improving the speed and efficiency of the fractionation.

Once the first-second bead pairs have been separated in step (c) the beads can be detached leaving pure modified polynucleotide molecules of formula A-P-B for subsequent use. In one embodiment this use comprises sequencing the polynucleotide moiety P using one of the direct methods known in the art including those taught in our earlier patent applications. However the method is equally useful in other applications where pure DNA or other nucleic acid samples are required.

It will be appreciated that if the first beads are magnetic it may not be necessary to adjust the density of the liquid medium in step (c); although this still may be beneficial. Rather, it may be possible to rely on applying a sufficiently strong magnetic field to overcome the inherent buoyancy of the first-second bead pairs. Thus, in one arrangement of the disclosure, there is provided a method of isolating a modified polynucleotide having the formula A-P-B from a crude mixture of modified polynucleotides also including those having the formulae A-P-A and B-P-B wherein P is a polynucleotide region and A and B are different modifying moieties or nucleotide sequences characterised by the steps of (a) reacting the crude mixture with magnetic first beads modified with complementary moieties A' capable of binding to A and non-magnetic second beads having a density lower than that of the first beads and modified with complementary moieties B' capable of binding to B to produce an intermediate product comprised of first-second bead-pairs connected by a modified polynucleotide together with one or more of unused or unpaired first and second beads and corresponding first-first, second-second bead-pairs; (b) separating the first-second bead-pairs and optionally any unused and/or unpaired second beads and/or second-second bead-pairs from the intermediate product in a liquid medium having a density greater than that of the first-second beads-pairs and (c) thereafter in an aqueous medium separating the first-second bead-pairs from the product of step (b) by applying a magnetic field sufficiently strong to overcome the buoyancy of the first-second bead pairs in the liquid medium.

The magnetic field may be used to simply hold the magnetic beads in a defined location allowing the other non-magnetic components to be simply washed away.

The liquid media used will preferably be aqueous media and in step (c) will preferably have a density less than that of the first-second bead-pairs but greater than that of the second beads; preferably 75-90% of the density of the first-second bead pairs.

It will also be appreciated that when the first beads are magnetic it is possible to first remove all of the magnetic components from the intermediate product magnetically and then separate the first-second beads from the magnetic components by density fractionation. Thus in a further arrangement of the disclosure there is provided a method of isolating a modified polynucleotide having the formula A-P-B from a crude mixture of modified polynucleotides also including those having the formulae A-P-A and B-P-B wherein P is a polynucleotide region and A and B are different modifying moieties or nucleotide sequences characterised by the steps of (a) reacting the crude mixture with magnetic first beads modified with complementary moieties A' capable of binding to A and non-magnetic second beads having a density lower than that of the first beads and modified with complementary moieties B' capable of binding to B to produce an intermediate product comprised of first-second bead-pairs connected by a modified polynucleotide together with one or more of unused or unpaired first and second beads and corresponding first-first, second-second bead-pairs; (b) separating the first-second bead pairs and optionally any unused and/or unpaired first beads and/or first-first bead pairs from the intermediate product in an liquid medium by the application of a magnetic field and (c) thereafter separating the first-second bead-pairs from the product of step (b) in a liquid medium having a density less than that of the first-second bead-pairs but greater than the second beads.

The liquid media employed will preferably be aqueous media and in step (c) will preferably have a density in the range 100-120% of the density of the first-second bead pairs.

The invention is now illustrated with reference to the following Example.

EXAMPLE

In this example the non-magnetic beads are polystyrene beads (density ~1.05) having a radius of 2$\mu$m, and functionalized with an anti-digoxigenin (Anti-DIG) antibody (Spherotech, product DIG-40-2). The magnetic beads (density

~1.8) have a radius of $0.5\mu$m and are functionalized with streptavidin (Dynal MyOne T1 streptavidin beads).

Polynucleotide fragments of 10kb were prepared and their 3' and 5' ends labelled with a mixture of digoxigenin (DIG) and biotin (BIO), such that each end of a given fragment is equally likely to carry either a DIG or a BIO moiety. Hence, the population of modified fragments contain approximately 25% DIG--, 25% BIO--, and 50% DIG--BIO (= BIO-DIG) fragments. This crude mixture of modified polynucleotides was then diluted to a concentration of $11pg/\mu$l, equivalent to approximately $10^6$ molecules/$\mu$l.

$5\mu$l of Dynal MyOne T1 beads (containing approximately $5 \times 10^7$ beads) were then washed into $200\mu$l of Dynal binding buffer according to the manufacturer's instructions, and $5\mu$l of the crude mixture (approximately $5 \times 10^6$ molecules) then added. This mixture was then placed on a tube rotator at 22°C for 30 minutes to allow beads to bind to any modified polynucleotides carrying BIO. The beads were then washed, following the manufacturer's instructions, to remove any unbound polynucleotide (specifically, molecules labelled at both ends with DIG which will not have bound to Dynal beads), and the beads then recovered into 10 microlitres of Link Buffer (1x Phosphate Buffered Saline, 0.1% Tween 20 detergent).

0.5ml of the non-magnetic Anti-DIG beads (approximately $5 \times 10^7$ beads) were washed by centrifugation into $10\mu$l of Link Buffer and added to a suspension of the washed magnetic beads (total volume = $20\mu$l). This mixture was again placed on a tube rotator at 22°C for 60 minutes to allow the non-magnetic beads to bind to any DNA polynucleotides carrying DIG.

At the end of this period a 130ul of wash buffer was added to the beads suspension and then it was further diluted with 280ul of a 1.9M sucrose solution (density 1.24), bringing the overall density of the solution to 1.17. The resulting mixture was then centrifuged at 800G for 30 minutes. This caused any used free Dynal beads (density 1.8) and any modified polynucleotide linking two Dynal beads to sink and those linked by one Dynal bead any non-magnetic bead to be driven to the top of the liquid. The sunk pellets were then removed by aspiration using a pipette, and discarded, leaving the upper part of the liquid undisturbed. Finally, the remaining upper layer of the liquid was removed and transferred to a new tube. The contents of this new tube comprised free non-magnetic beads and Dynal/Anti-DIG bead pairs linked by a modified polynucleotide having one BIO and one DIG end.

A magnetic field was then applied to a wall of the new tube for 1 minute causing the Dynal/Anti-DIG bead pairs to be drawn to the wall and thus separated. Thereafter, the remaining liquid was aspirated out of the tube, and the magnetic field removed. $500\mu$l of wash buffer (10mM Tris HCl (pH7.5), 1mM EDTA) was then added, and the remaining bead-pairs gently re-suspended. The magnetic field was then re-applied for a further one minute to once again draw the Dynal/Anti-DIG bead pairs to the wall of the tube before the liquid was again aspirated away and the magnetic field removed. Finally, the bead-pairs were recovered and re-suspended in $100\mu$l of wash buffer prior to the beads being removed from the polynucleotide.

Further methods of effecting the separation are set out schematically in the Figure.

## Claims

1. A method of isolating a modified polynucleotide having the formula A-P-B from a crude mixture of modified polynucleotides also including those having the formulae A-P-A and B-P-B, wherein P is a polynucleotide region and A and B are different modifying moieties or nucleotide sequences, **characterised by** the steps of (a) reacting the crude mixture with first beads modified with complementary moieties A' capable of binding to A and second beads having a density lower than that of the first beads and modified with complementary moieties B' capable of binding to B to produce an intermediate product comprised of first-second bead-pairs connected by a modified polynucleotide together with one or more of unused or unpaired first and second beads and corresponding first-first, second-second bead-pairs; (b) separating the first-second bead-pairs and optionally any unused or unpaired second beads or second-second bead-pairs from the intermediate product in an aqueous medium having a density greater than that of the first-second beads-pairs but less than that of the first beads and (c) thereafter separating the first-second bead-pairs from the product of step (b) in an aqueous medium having a density less than that of the first-second bead-pairs but greater than that of the second beads.

2. A method as claimed in claim 1 **characterised in that** the first beads are magnetic.

3. A method as claimed in any of the preceding claims **characterised in that** step (a) comprises two sub-steps in which the crude mixture is either first treated with the first beads and thereafter the second beads or first treated with the second beads and thereafter the first beads.

4. A method as claimed in any of the preceding claims **characterised in that** the densities of the liquid media are controlled or modified by use of a Group IA or IIA soluble salt or a sugar.

**5.** A method as claimed in any of the preceding claims **characterised in that** the density of the liquid medium in step (b) is 100-120% of the density of the first-second bead pairs.

**6.** A method as claimed in any of any of the preceding claims **characterised in that** the density of the liquid medium in step (c) is 75-90% of the density of the first-second bead pairs.

**7.** A method as claimed in any one of the preceding claims **characterised in that** the first beads have a density of greater than 1.5 g/cm$^3$ and the second beads have a density of less than 1.5 g/cm$^3$

**8.** A method as claimed in any of the preceding claims characterised that at least one of the pairs A and A' or B and B' are selected from avidin/biotin, streptavidin/biotin, a protein/antibody pair or two complementary oligonucleotide regions.

**9.** A method as claimed in any of the preceding claims **characterised by** further comprising step (d) detaching the beads from the first-second bead pair.

**10.** A method as claimed in any of the preceding claims **characterised in that** the modified polynucleotide or at least the P moiety thereof is a ribonucleotide, a deoxyribonucleotide or a synthetic analogue thereof.

**11.** Use of a method as claimed in any of the preceding claims to isolate a polynucleotide for subsequent sequencing.

**Patentansprüche**

**1.** Verfahren zum Isolieren eines modifizierten Polynukleotids mit der Formel A-P-B aus einem Rohgemisch von modifizierten Polynukleotiden, das auch solche mit den Formeln A-P-A und B-P-B einschließt, wobei P eine Polynukleotidregion ist und A und B verschiedene modifizierende Einheiten oder Nukleotidsequenzen sind, **gekennzeichnet durch** die Schritte (a) Umsetzen des Rohgemisches mit ersten Kügelchen, die mit komplementären Einheiten A' modifiziert sind, die zum Binden an A in der Lage sind, und zweiten Kügelchen, die eine geringere Dichte als die der ersten Kügelchen aufweisen und mit komplementären Einheiten B' modifiziert sind, die zum Binden an B in der Lage sind, um ein Zwischenprodukt herzustellen, aufgebaut aus erstes-zweites-Kügelchen-Paaren, die verbunden sind durch ein modifiziertes Polynukleotid, zusammen mit einem oder mehreren von nicht-verwendeten oder nicht-gepaarten ersten und zweiten Kügelchen und entsprechenden erstes-erstes- und zweites-zweites-Kügelchen-Paaren; (b) Abtrennen der erstes-zweites Kügelchen-Paare und gegebenenfalls jedweder nicht-verwendeter oder nicht-gepaarter zweiter Kügelchen oder zweites-zweites-Kügelchen-Paare von dem Zwischenprodukt in einem wässrigen Medium, aufweisend eine Dichte, die größer ist als die der erstes-zweites-Kügelchen-Paare, aber geringer ist als die der ersten Kügelchen, und (c) danach Abtrennen der erstes-zweites-Kügelchen-Paare von dem Produkt aus Schritt (b) in einem wässrigen Medium, aufweisend eine Dichte, die geringer als die der erstes-zweites-Kügelchen-Paare ist, aber größer ist als die der zweiten Kügelchen.

**2.** Verfahren, wie in Anspruch 1 beansprucht, **dadurch gekennzeichnet, dass** die ersten Kügelchen magnetisch sind.

**3.** Verfahren, wie in beliebigen der vorhergehenden Ansprüche beansprucht, **dadurch gekennzeichnet, dass** Schritt (a) zwei Unterschritte umfasst, in denen das Rohgemisch entweder zuerst mit den ersten Kügelchen und danach den zweiten Kügelchen behandelt wird oder zuerst mit den zweiten Kügelchen und danach den ersten Kügelchen behandelt wird.

**4.** Verfahren, wie in beliebigen der vorhergehenden Ansprüche beansprucht, **dadurch gekennzeichnet, dass** die Dichten der flüssigen Medien durch Verwendung eines löslichen Salzes der Gruppe IA oder IIA oder Zucker gesteuert oder modifiziert werden.

**5.** Verfahren, wie in beliebigen der vorhergehenden Ansprüche beansprucht, **dadurch gekennzeichnet, dass** die Dichte des flüssigen Mediums in Schritt (b) 100-120% der Dichte der erstes-zweites-Kügelchen-Paare beträgt.

**6.** Verfahren, wie in beliebigen der vorhergehenden Ansprüche beansprucht, **dadurch gekennzeichnet, dass** die Dichte des flüssigen Mediums in Schritt (c) 75-90% der Dichte der erstes-zweites-Kügelchen-Paare beträgt.

**7.** Verfahren, wie in einem beliebigen der vorhergehenden Ansprüche beansprucht, **dadurch gekennzeichnet, dass** die ersten Kügelchen eine Dichte von mehr als 1,5 g/cm$^3$ aufweisen und die zweiten Kügelchen eine Dichte von weniger als 1,5 g/cm$^3$ aufweisen.

8. Verfahren, wie in beliebigen der vorhergehenden Ansprüche beansprucht, **dadurch gekennzeichnet, dass** mindestens eines der Paare A und A' oder B und B' ausgewählt ist aus Avidin/Biotin, Streptavidin/Biotin, einem Protein/Antikörper-Paar oder zwei komplementären Oligonukleotidregionen.

9. Verfahren, wie in beliebigen der vorhergehenden Ansprüche beansprucht, **dadurch gekennzeichnet, dass** es ferner den Schritt (d) des Ablösens der Kügelchen aus dem erstes-zweites-Kügelchen-Paar umfasst.

10. Verfahren, wie in beliebigen der vorhergehenden Ansprüche beansprucht, **dadurch gekennzeichnet, dass** das modifizierte Polynukleotid oder zumindest die P-Einheit davon ein Ribonukleotid, ein Desoxyribonukleotid oder ein synthetisches Analogon davon ist.

11. Verwendung eines Verfahrens, wie in beliebigen der vorhergehenden Ansprüche beansprucht, zum Isolieren eines Polynukleotids für die anschließende Sequenzierung.


**Revendications**

1. Procédé d'isolement d'un polynucléotide modifié ayant la formule A-P-B à partir d'un mélange brut de polynucléotides modifiés comprenant en outre ceux ayant les formules A-P-A et B-P-B, dans lequel P est une région polynucléotidique et A et B sont des fragments ou séquences nucléotidiques de modification différents, **caractérisé par** les étapes de (a) réaction du mélange brut avec des premières billes modifiées avec des fragments complémentaires A' capables de se lier à A et des deuxièmes billes ayant une masse volumique inférieure à celle des premières billes et modifiées avec des fragments complémentaires B' capables de se lier à B pour produire un produit intermédiaire constitué de paires de première-deuxième billes reliées par un polynucléotide modifié conjointement avec une ou plusieurs des premières et deuxièmes billes inutilisées ou non appariées et des paires de première-première, deuxième-deuxième billes correspondantes ; (b) séparation des paires de première-deuxième billes et facultativement des deuxièmes billes inutilisées ou non appariées ou des paires de deuxième-deuxième billes du produit intermédiaire dans un milieu aqueux ayant une masse volumique supérieure à celle des paires de première-deuxième billes mais inférieure à celle des premières billes et (c) ensuite, séparation des paires de première-deuxième billes du produit de l'étape (b) dans un milieu aqueux ayant une masse volumique inférieure à celle des paires de première-deuxième billes mais supérieure à celle des deuxièmes billes.

2. Procédé selon la revendication 1 **caractérisé en ce que** les premières billes sont magnétiques.

3. Procédé selon l'une quelconque des revendications précédentes, **caractérisé en ce que** l'étape (a) comprend deux sous-étapes dans lesquelles le mélange brut est soit traité dans un premier temps avec les premières billes puis les deuxièmes billes ou traité dans un premier temps avec les deuxièmes billes puis avec les premières billes.

4. Procédé selon l'une quelconque des revendications précédentes, **caractérisé en ce que** les masses volumiques des milieux liquides sont contrôlées ou modifiées au moyen d'un sel soluble d'élément du groupe IA ou IIA ou d'un sucre.

5. Procédé selon l'une quelconque des revendications précédentes, **caractérisé en ce que** la masse volumique du milieu liquide medium dans l'étape (b) est de 100 à 120 % de la masse volumique des paires de première-deuxième billes.

6. Procédé selon l'une quelconque des revendications précédentes, **caractérisé en ce que** la masse volumique du milieu liquide dans l'étape (c) est de 75 à 90 % de la masse volumique des paires de première-deuxième billes.

7. Procédé selon l'une quelconque des revendications précédentes, **caractérisé en ce que** les premières billes ont une masse volumique supérieure à 1,5 g/cm$^3$ et les deuxièmes billes ont une masse volumique inférieure à 1,5 g/cm$^3$.

8. Procédé selon l'une quelconque des revendications précédentes, **caractérisé en ce qu'**au moins une des paires A et A' ou B et B' sont choisies parmi avidine/biotine, streptavidine/biotine, une paire protéine/anticorps ou deux régions oligonucléotidiques complémentaires.

**9.** Procédé selon l'une quelconque des revendications précédentes, **caractérisé en ce qu'**il comprend en outre l'étape (d) de détachement des billes de la paire de première-deuxième billes.

**10.** Procédé selon l'une quelconque des revendications précédentes, **caractérisé en ce que** le polynucléotide modifié ou au moins le fragment P de celui-ci est un ribonucléotide, un désoxyribonucléotide ou un analogue synthétique de ceux-ci.

**11.** Utilisation d'un procédé selon l'une quelconque des revendications précédentes pour isoler un polynucléotide pour séquençage consécutif.

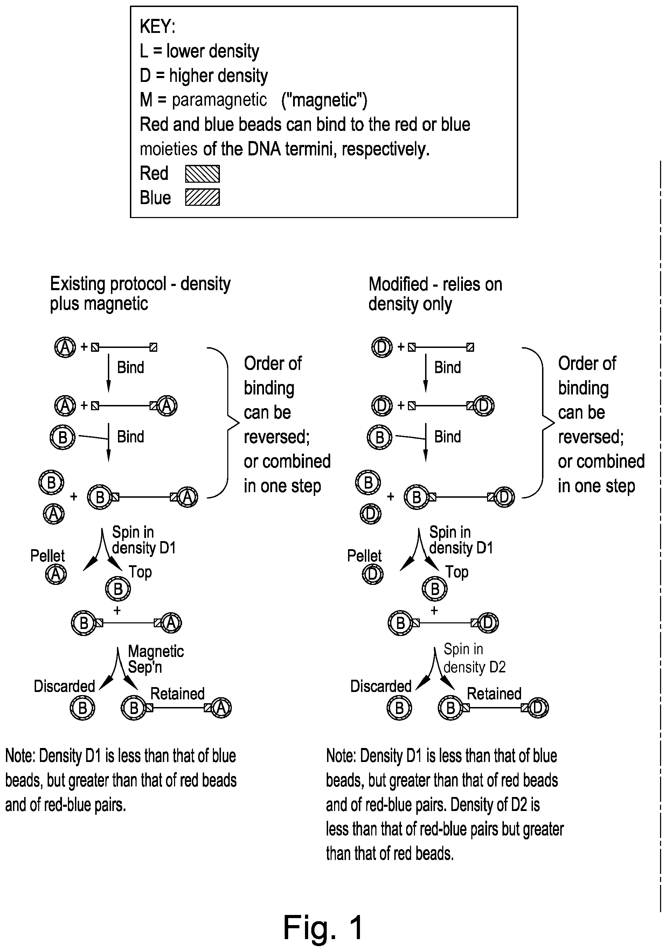

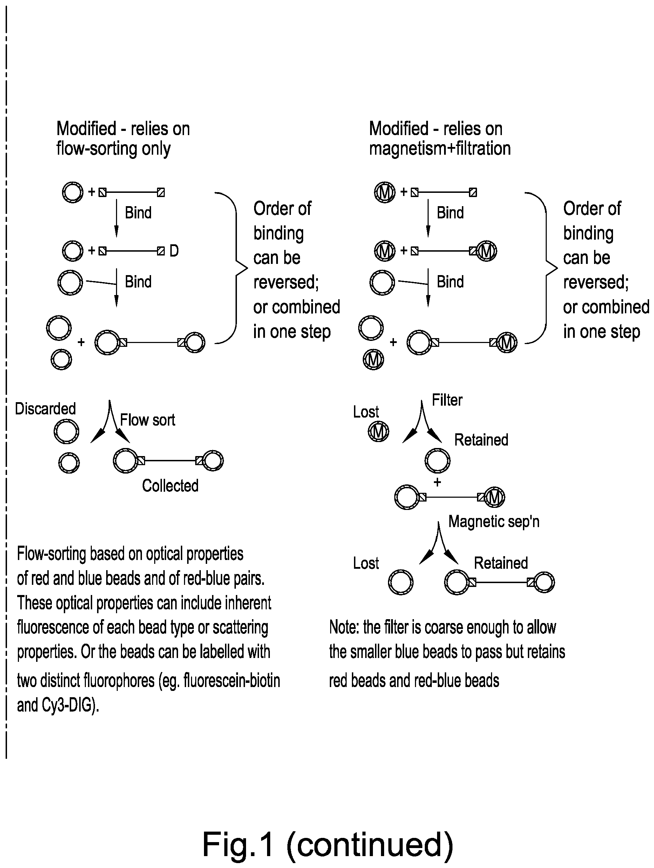

Fig. 1

Modified - relies on
flow-sorting only

Modified - relies on
magnetism+filtration

Order of
binding
can be
reversed;
or combined
in one step

Flow-sorting based on optical properties
of red and blue beads and of red-blue pairs.
These optical properties can include inherent
fluorescence of each bead type or scattering
properties. Or the beads can be labelled with
two distinct fluorophores (eg. fluorescein-biotin
and Cy3-DIG).

Note: the filter is coarse enough to allow
the smaller blue beads to pass but retains
red beads and red-blue beads

# Fig.1 (continued)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0127328 A
- US 2014335528 A

- EP 2196544 A

**Non-patent literature cited in the description**

- *J. Nanotechnol.,* 2016, vol. 7, 138-148